# FASCICULE DE BREVET EUROPEEN

(11) **EP 3 755 199 B1**
(45) Date de publication et mention de la délivrance du brevet: **30.03.2022**
(21) Numéro de dépôt: 19706278.9
(22) Date de dépôt: 21.02.2019
(51) Int. Cl.: A61B 3/00, A61B 3/036

(54) **PROCÉDÉ DE TEST VISUEL, MODULE DE COMMANDE ET SYSTEM ASSOCIÉS**
SEHTESTVERFAHREN, ENTSPRECHENDES STEUERMODUL UND SYSTEM
EYESIGHT TESTING METHOD, ASSOCIATED CONTROL MODULE AND SYSTEM

(30) Priorité: 23.02.2018 FR 1851616
(43) Date de publication de la demande: 30.12.2020
(73) Titulaire: SIVIEW, 91460 Marcoussis (FR)
(72) Inventeur: GUILLEMAIN, Nathalie, 95320 Saint Leu la Forêt (FR); PICHEREAU, Laure, 91460 Marcoussis (FR)
(74) Mandataire: Cabinet Camus Lebkiri
(86) Numéro de dépôt international: PCT/EP2019/054362
(87) Numéro de publication internationale: WO 2019/162401

(56) Documents cités:
- WO-A1-02/00105
- JP-A- 2007 267 822
- US-A1- 2005 264 760
- US-A1- 2016 317 025

## Description

### DOMAINE TECHNIQUE DE L'INVENTION

Le domaine de l'invention est celui des examens de vue qui, pratiqués sur un sujet, permettent de déterminer s'il a besoin d'une correction et laquelle.

L'invention se rapporte plus particulièrement à un procédé de test visuel par exemple lors d'un examen de vue, au moyen d'un support qui est soit une tête de réfracteur, automatique ou manuelle, soit une paire de lunettes d'essai, permettant de sélectionner le cas échéant au moins une direction parmi une pluralité de directions. L'invention concerne également un module de commande pour la mise en œuvre du procédé de test visuel ainsi qu'un système comportant le module de commande, un écran de sélection et un écran de lecture pour la mise en œuvre du procédé de test visuel.

### ETAT DE LA TECHNIQUE ANTERIEURE

Un examen de vue d'un sujet débute par une mesure de réfraction objective et se poursuit par une mesure de réfraction subjective.

La mesure de réfraction objective est généralement réalisée au moyen d'un autoréfractomètre. Alternativement à l'utilisation d'un autoréfractomètre, une technique manuelle de skiascopie est parfois utilisée. A défaut l'opérateur peut partir de la précédente correction du sujet examiné.

Lors de la mesure de réfraction subjective, l'opérateur fait tester différentes corrections visuelles au sujet examiné au moyen d'un matériel adapté qui peut être :
- une paire de lunettes d'essai avec des verres d'essai,
- une tête de réfracteur manuelle ou
- une tête de réfracteur automatique.
Dans le premier cas, l'opérateur change manuellement les verres d'essai de la paire de lunettes d'essai. Avec une tête de réfracteur, l'opérateur fait défiler différents verres devant les yeux du sujet examiné, au moyen de mollettes dans le cas d'une tête de réfracteur manuelle ou via une console de commande dans le cas d'une tête de réfracteur automatique.

Au cours de la mesure de réfraction subjective, généralement dans le cadre d'une recherche d'astigmatisme, l'opérateur peut mettre en œuvre un test dit « test de Parent » selon lequel un verre optique est agencé devant un œil du sujet examiné pendant que ce dernier observe un écran de lecture sur lequel un cadran, dit « cadran de Parent », est affiché. Le test de Parent est un test de dépistage d'astigmatisme qui ne permet pas de définir avec précision une valeur d'astigmatisme. Si un astigmatisme est mis en évidence par un test de Parent, d'autres tests sont requis pour déterminer précisément l'astigmatisme détecté. Le test de Parent est réalisé sur un sujet « brouillé », c'est-à-dire rendu artificiellement légèrement myope afin d'éviter tout effet perturbateur de l'accommodation. Par ailleurs, le test de Parent est réalisé en monoculaire, c'est-à-dire sur un seul œil. Il peut être réalisé sur un œil puis l'autre ou bien sur un seul des deux yeux.

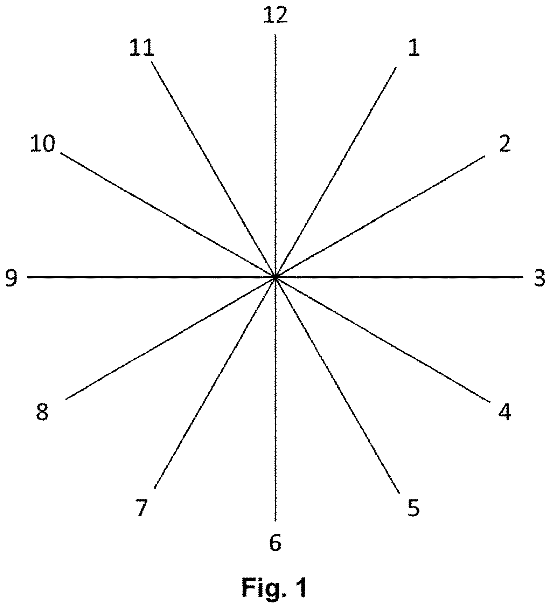

La figure 1 représente schématiquement un tel cadran. Le cadran présente douze demi-droites, de même longueur et de même origine, définissant douze secteurs angulaires identiques de 30°. Les demi-droites sont numérotées de 1 à 12 dans le sens des aiguilles d'une montre par un chiffre placé à leur extrémité, à la manière d'un cadran de montre ou d'horloge. Les douze demi-droites matérialisent six directions qui peuvent être identifiées de la manière suivante à partir des numéros à leurs extrémités :
- première direction 1-7,
- deuxième direction 2-8,
- troisième direction 3-9,
- quatrième direction 4-10,
- cinquième direction 5-11,
- sixième direction 6-12.

Le sujet examiné doit alors dire si l'une des directions lui apparaît mieux, plus nette que les autres. Le cas échéant, il identifie une telle direction au moyen d'au moins un numéro à une extrémité. Par exemple, la première direction 1-7 peut être identifiée grâce au 1 et/ou grâce au 7.

A partir d'une direction choisie par le sujet examiné sur l'écran de lecture, l'opérateur calcule une direction sur la rétine du sujet examiné puis un axe de cylindre compensateur.

Si deux directions contiguës lui apparaissent toutes les deux mieux, plus nettes que les autres, le sujet examiné les identifie toutes les deux, toujours au moyen d'au moins un numéro à une extrémité, puis l'opérateur travaille à partir de la bissectrice de ces deux directions contiguës.

Il existe un risque d'erreur par l'opérateur dans la transcription de l'information donnée par le sujet examiné, notamment du fait qu'une même direction peut être identifiée de plusieurs manières différentes par le sujet examiné (« direction 1-7 », « direction à 1h », ...). S'il a un doute, l'opérateur peut vérifier sa compréhension auprès du sujet examiné. En revanche, le sujet examiné ne peut pas vérifier que la transcription de l'opérateur est correcte. Dans le même temps, un tel test n'est généralement qu'une étape d'un examen de vue qui en comporte de nombreuses autres et il est souhaité de pouvoir aller vite. Par ailleurs, en cas d'erreur, l'examen de vue risque de ne pas aboutir ou de mettre plus longtemps à aboutir ou d'aboutir à un résultat erroné.

Le document WO 02/00105 divulgue un procédé de test visuel selon le préambule de la revendication 1 et un système selon le préambule de la revendication 7.

Il est donc recherché une solution permettant, dans le cadre d'un examen de vue, de sélectionner le cas échéant au moins une direction parmi une pluralité de directions rapidement et sans se tromper sur la ou les directions retenues.

### EXPOSE DE L'INVENTION

Dans ce contexte, l'invention vise à remédier à tout ou partie des inconvénients de l'état de la technique identifiés ci-dessus.

A cette fin, un premier aspect de l'invention concerne un procédé de test visuel comportant une étape d'agencement d'un verre optique dans un emplacement d'un support et les étapes suivantes selon lesquelles un module de commande :
- affiche sur un écran de sélection et sur un écran de lecture une pluralité de lignes, chaque ligne s'étendant dans une direction distincte, l'écran de sélection et l'écran de lecture étant deux écrans distincts ou alternativement un seul et même écran ;
- reçoit une sélection d'une ligne sur l'écran de sélection ;
le procédé étant tel que :
- lorsque le module de commande reçoit une sélection d'une première ligne alors qu'aucune autre ligne n'est déjà sélectionnée, le module de commande sélectionne ladite première ligne en modifiant au moins une caractéristique d'aspect de ladite première ligne de manière qu'elle passe d'un aspect initial à un aspect modifié qui la différencie des lignes non sélectionnées ;
- lorsque le module de commande reçoit une sélection d'une nouvelle ligne alors qu'au moins une autre ligne est déjà sélectionnée, le module de commande sélectionne ladite nouvelle ligne en modifiant au moins une caractéristique d'aspect de ladite nouvelle ligne de manière qu'elle passe d'un aspect initial à un aspect modifié qui la différencie des lignes non sélectionnées, et le module de commande désélectionne en lui redonnant son aspect initial chaque ligne qui n'est pas directement contiguë à ladite nouvelle ligne sélectionnée.

Un deuxième aspect de l'invention concerne un module de commande pour la mise en œuvre du procédé de test visuel selon le premier aspect de l'invention, le module de commande ayant :
- des moyens pour afficher sur un écran de sélection et sur un écran de lecture une pluralité de lignes, chaque ligne s'étendant dans une direction distincte, l'écran de sélection et l'écran de lecture étant deux écrans distincts ou alternativement un seul et même écran ;
- des moyens pour recevoir une sélection d'une ligne de la pluralité de lignes sur l'écran de sélection ;
- des moyens pour recevoir une commande spécifique mettant fin au procédé de test visuel ;
   les moyens d'affichage sur l'écran de sélection et l'écran de lecture et de réception d'une sélection sur l'écran de sélection étant tels que :
- lorsque le module de commande reçoit une sélection d'une première ligne alors qu'aucune autre ligne n'est déjà sélectionnée, le module de commande sélectionne ladite première ligne en modifiant au moins une caractéristique d'aspect de ladite première ligne de manière qu'elle passe d'un aspect initial à un aspect modifié qui la différencie des lignes non sélectionnées ;
- lorsque le module de commande reçoit une sélection d'une nouvelle ligne alors qu'au moins une autre ligne est déjà sélectionnée, le module de commande sélectionne ladite nouvelle ligne en modifiant au moins une caractéristique d'aspect de ladite nouvelle ligne de manière qu'elle passe d'un aspect initial à un aspect modifié qui la différencie des lignes non sélectionnées, et le module de commande désélectionne en lui redonnant son aspect initial chaque ligne qui n'est pas directement contiguë à ladite nouvelle ligne sélectionnée.

Un troisième aspect de l'invention concerne un système pour la mise en œuvre d'un procédé de test visuel selon le premier aspect de l'invention, comportant le module de commande selon le deuxième aspect de l'invention ainsi que l'écran de sélection et l'écran de lecture.

Outre les caractéristiques qui viennent d'être mentionnées dans les paragraphes précédents, le procédé de test visuel selon le premier aspect de l'invention, le module de commande selon le deuxième aspect de l'invention et le système de commande selon le troisième aspect de l'invention peuvent présenter une ou plusieurs caractéristiques complémentaires parmi les suivantes, considérées individuellement ou selon toutes les combinaisons techniquement possibles :
- Lorsque le module de commande reçoit une sélection d'une ligne qui est déjà sélectionnée, le module de commande désélectionne ladite ligne en lui redonnant son aspect initial.
- Lorsque, des première et deuxième lignes étant déjà sélectionnées, le module de commande reçoit une sélection d'une nouvelle ligne qui est une troisième ligne, le module de commande :
   ∘ sélectionne ladite troisième ligne en modifiant au moins une caractéristique d'aspect de ladite troisième ligne de manière qu'elle passe d'un aspect initial à un aspect modifié qui la différencie des lignes non sélectionnées, et
   ∘ désélectionne lesdites première et deuxième lignes en leur redonnant leur aspect initial.
- Le module de commande affiche sur l'écran de sélection un symbole de validation en même temps que la pluralité de lignes.
- Le symbole de validation est un premier symbole de validation tant qu'aucune ligne n'est sélectionnée, un deuxième symbole de validation distinct du premier symbole de validation lorsqu'une seule ligne est sélectionnée et un troisième symbole de validation distinct des premier et deuxième symboles de validation lorsque deux lignes sont sélectionnées.
- Le module de commande calcule une valeur d'axe de cylindre compensateur à partir de la ou des lignes sélectionnée(s) et affiche cette valeur sur l'écran de sélection ou alternativement fournit cette valeur en entrée d'un algorithme.
- L'écran de sélection et l'écran de lecture sont deux écrans distincts.
- Alternativement, l'écran de sélection et l'écran de lecture sont un seul et même écran.

### BREVE DESCRIPTION DES FIGURES

D'autres caractéristiques et avantages de l'invention ressortiront à la lecture de la description qui suit, en référence aux figures annexées.
- La figure 1, déjà décrite, montre un cadran de Parent classique.
- La figure 2a montre un diagramme des étapes d'une première alternative du procédé de test visuel selon le premier aspect de l'invention.
- La figure 2b montre un diagramme des étapes d'une deuxième alternative du procédé de test visuel selon le premier aspect de l'invention.
- La figure 3 montre un agencement d'un verre optique dans un support, selon une première étape du procédé de test visuel selon le premier aspect de l'invention.
- La figure 4a montre un premier exemple d'affichage, seulement sur un écran de sélection ou à la fois sur un écran de sélection et un écran de lecture, d'une pluralité de lignes et d'un symbole de validation.
- La figure 4b montre un deuxième exemple d'affichage, seulement sur un écran de sélection ou à la fois sur un écran de sélection et un écran de lecture, d'une pluralité de lignes et d'un symbole de validation.
- La figure 4c montre un troisième exemple d'affichage, seulement sur un écran de sélection ou à la fois sur un écran de sélection et un écran de lecture, d'une pluralité de lignes et d'un symbole de validation.
- La figure 4d montre un quatrième exemple d'affichage, seulement sur un écran de sélection ou à la fois sur un écran de sélection et un écran de lecture, d'une pluralité de lignes et d'un symbole de validation.
- La figure 4e montre un cinquième exemple d'affichage, seulement sur un écran de sélection ou à la fois sur un écran de sélection et un écran de lecture, d'une pluralité de lignes et d'un symbole de validation.

Pour plus de clarté, des éléments identiques ou similaires sont repérés par des signes de référence identiques sur l'ensemble des figures.

### DESCRIPTION DETAILLEE D'AU MOINS UN MODE DE REALISATION DE L'INVENTION

Un premier aspect de l'invention concerne un procédé de test visuel au moyen d'un module de commande, permettant de sélectionner au moins une direction parmi une pluralité de directions. Un deuxième aspect de l'invention concerne le module de commande pour la mise en œuvre du procédé de test visuel selon le premier aspect de l'invention. Un troisième aspect de l'invention concerne un système comportant le module de commande selon le deuxième aspect de l'invention, un écran de sélection et un écran de lecture, pour la mise en œuvre du procédé de test visuel selon le premier aspect de l'invention.

Dans le cadre de la présente invention, on entend par « verre optique » un élément optique de qualité adaptée à la réalisation de lunettes de vue, sans préjuger du matériau dans lequel il est réalisé et qui peut notamment être du verre ou du plastique. Les termes « verre ophtalmique » ou « verre correcteur » peuvent également être employés. Le terme « lentille optique » peut également être employé en toute rigueur, à condition de bien l'interpréter au sens d'un élément optique de qualité adaptée à la réalisation de lunettes de vue, par opposition à une lentille cornéenne ou lentille de contact.

### Procédé selon le premier aspect de l'invention

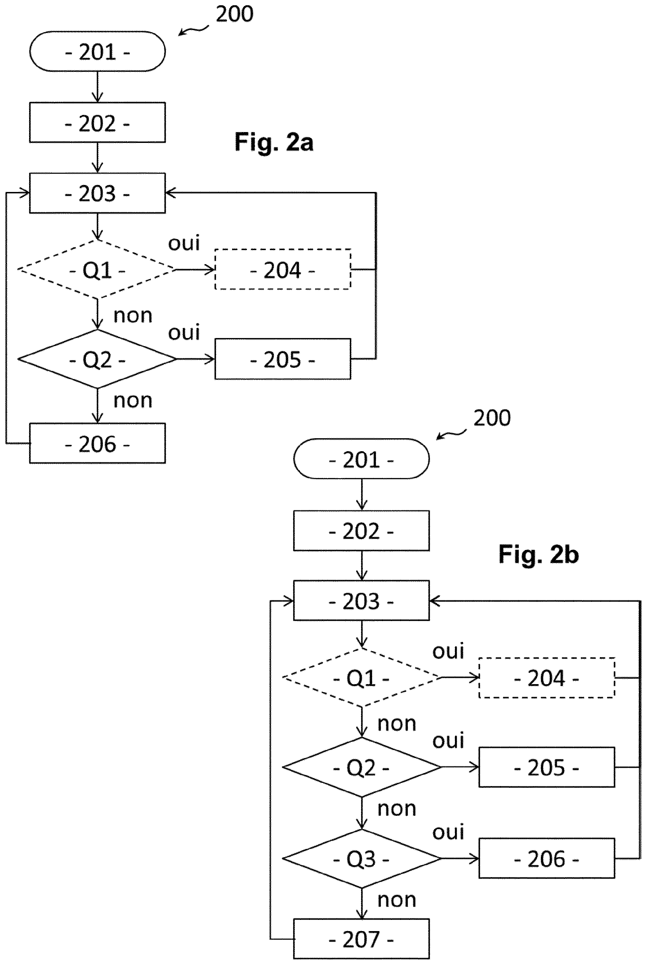

La figure 2a montre un diagramme des étapes du procédé 200 selon une première alternative. La figure 2b montre un diagramme des étapes du procédé 200 selon une deuxième alternative.

Dans la suite, on entend par « un opérateur sélectionne une ligne » le fait que l'opérateur réalise une action de sélection de ladite ligne, en interaction avec l'écran de sélection. On entend par « le module de commande sélectionne une ligne » le fait que le module de commande valide la sélection reçue de l'opérateur et modifie au moins une caractéristique d'aspect de ladite ligne de manière qu'elle passe d'un aspect initial à un aspect modifié qui la différencie des lignes non sélectionnées. On entend par « le module de commande désélectionne une ligne » le fait que le module de commande modifie ladite ligne de manière qu'elle perd son aspect modifié et reprend son aspect initial. On entend par « ligne sélectionnée » une ligne sélectionnée à la fois par l'opérateur et par le module de commande, c'est-à-dire une ligne pour laquelle l'action de sélection de l'opérateur est validée par le module de commande. On entend par « ligne non sélectionnée » une ligne non sélectionnée par l'opérateur (pas d'action de sélection), ou une ligne sélectionnée par l'opérateur mais non sélectionnée par le module de commande c'est-à-dire pour laquelle l'opération de sélection de l'opérateur n'est pas validée par le module de commande ou est interprétée par le module de commande comme une désélection.

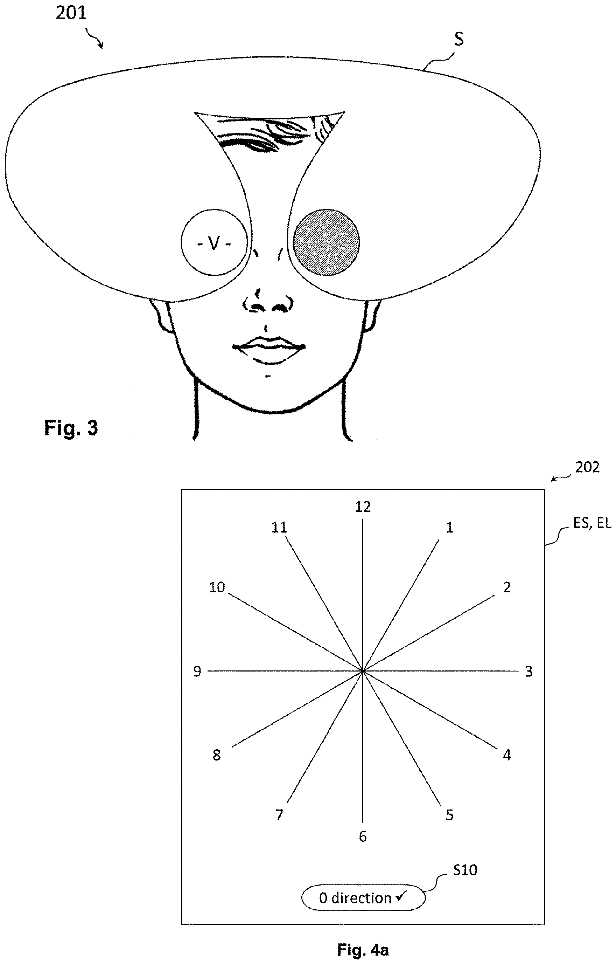

La première alternative de la figure 2a est à présent décrite. Selon une première étape 201 illustrée à la figure 3, un verre optique V est agencé dans un emplacement d'un support S qui est soit une tête de réfracteur, automatique ou manuelle, soit une paire de lunettes d'essai. Une tête de réfracteur ou une paire de lunettes d'essai sont tous les deux un support comportant des premier et second emplacements, un pour chaque œil, pouvant chacun recevoir un élément qui est soit un cache opaque, soit un verre optique. Dans le cas d'une paire de lunettes d'essai, l'opérateur manipule et agence manuellement les différents éléments dans les emplacements du support. Dans le cas d'une tête de réfracteur, l'opérateur ne manipule pas directement les différents éléments : il les agence dans les différents éléments du support en actionnant des mollettes dans le cas d'une tête de réfracteur manuelle, ou via une console de commande dans le cas d'une tête de réfracteur automatique. Le support S est préférentiellement une tête de réfracteur automatique et dans ce cas, c'est le module de commande selon le deuxième aspect de l'invention qui assure la fonction de « console de commande » de la tête de réfracteur automatique.

Dans le cadre de la présente invention, on entend par « verre optique agencé dans un emplacement du support » soit un unique verre optique, soit une combinaison de plusieurs verres optiques, permettant de réaliser une certaine correction optique. Le support S est destiné à être placé devant le sujet examiné de manière que le sujet examiné regarde, à travers les premier et second emplacements, un écran de lecture sur lequel une pluralité de lignes est affichée, chaque ligne s'étendant dans une direction distincte. Une étape d'agencement d'un élément dans un emplacement peut commencer le cas échéant par une étape de retrait d'un précédent élément dudit emplacement.

Dans le cadre du procédé de test visuel selon le premier aspect de l'invention, le support S est utilisée en monoculaire œil droit ou en monoculaire œil gauche mais pas en binoculaire. En monoculaire œil droit, un verre optique est agencé dans l'emplacement correspondant à l'œil droit tandis qu'un cache est agencé dans l'emplacement correspondant à l'œil gauche. En monoculaire œil gauche, un verre optique est agencé dans l'emplacement correspondant à l'œil gauche tandis qu'un cache est agencé dans l'emplacement correspondant à l'œil droit.

Selon une deuxième étape 202 illustrée par exemple à la figure 4a, le module de commande affiche sur un écran de sélection ES et sur un écran de lecture EL, qui sont deux écrans distincts ou alternativement un seul et même écran, une pluralité de lignes, chaque ligne s'étendant dans une direction distincte. La pluralité de lignes comporte typiquement douze demi-droites de même origine, définissant douze secteurs angulaires identiques de 30°. Les demi-droites sont numérotées de 1 à 12 dans le sens des aiguilles d'une montre par un chiffre placé à leur extrémité, à la manière d'un cadran de montre ou d'horloge. Les douze demi-droites matérialisent six directions qui peuvent être identifiées de la manière suivante à partir des numéros à leurs extrémités :
- première direction 1-7,
- deuxième direction 2-8,
- troisième direction 3-9,
- quatrième direction 4-10,
- cinquième direction 5-11,
- sixième direction 6-12.
Plus généralement, la pluralité de lignes peut comporter de huit à vingt-quatre demi-droites de même origine, définissant respectivement de huit secteurs angulaires identiques de 45° à vingt-quatre secteurs angulaires identiques de 15°. Toutes les demi-droites sont typiquement de même longueur.

Lors de la deuxième étape 202, aucune ligne n'est sélectionnée et toutes les lignes présentent préférentiellement un même aspect initial. Selon un mode préférentiel de l'invention, le module de commande affiche, au moins sur l'écran de sélection et préférentiellement à la fois sur l'écran de sélection ES et sur l'écran de lecture EL, un premier symbole de validation S10 lorsqu'aucune ligne n'est sélectionnée, le premier symbole de validation S10 indiquant qu'aucune ligne n'est sélectionnée et/ou qu'aucune direction n'est préférée.

Selon une troisième étape 203, l'opérateur sélectionne sur l'écran de sélection ES une ligne de la pluralité de lignes et le module de commande reçoit la sélection de ladite ligne. Le procédé 200 se poursuit ensuite préférentiellement par une première décision Q1 ou directement par une deuxième décision Q2. Selon le mode préférentiel de l'invention, le module de commande affiche, au moins sur l'écran de sélection ES et préférentiellement à la fois sur l'écran de sélection ES et sur l'écran de lecture EL :
- un deuxième symbole de validation S11 lorsqu'une et une seule ligne est sélectionnée, le deuxième symbole de validation S11 indiquant qu'une ligne est sélectionnée et/ou qu'une direction est préférée ;
- un troisième symbole de validation S12 lorsque deux lignes sont sélectionnées, le troisième symbole de validation S12 indiquant que deux lignes sont sélectionnées et/ou que deux directions sont préférées.

Selon la première décision Q1, le module de commande répond à la question : « la ligne sélectionnée par l'opérateur est-elle actuellement déjà sélectionnée par le module de commande ? ». Si oui, selon une quatrième étape 204 préférentielle, le module de commande désélectionne ladite ligne. Le procédé 200 peut ensuite soit continuer en rebouclant sur la troisième étape 203, soit se terminer. Si la ligne sélectionnée par l'opérateur n'était pas déjà sélectionnée par le module de commande, le procédé 200 se poursuit par la deuxième décision Q2.

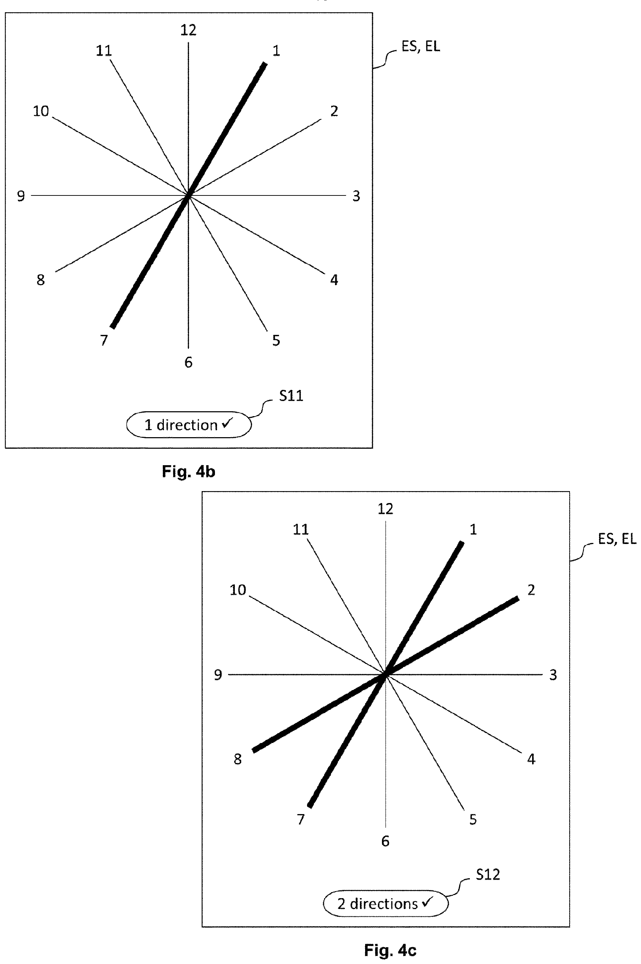

Selon la deuxième décision Q2, le module de commande répond à la question : « la ligne sélectionnée par l'opérateur est-elle une première ligne ? ». Si oui, c'est-à-dire si aucune ligne n'est déjà sélectionnée par le module de commande, selon une cinquième étape 205 illustrée par exemple à la figure 4b, le module de commande sélectionne ladite première ligne. Dans l'exemple de la figure 4b, la première ligne sélectionnée est la première direction 1-7 Le procédé 200 peut ensuite soit continuer en rebouclant sur la troisième étape 203, soit se terminer.

Si la ligne sélectionnée par l'opérateur, dite « nouvelle ligne », n'est pas la première ligne, c'est-à-dire si au moins une autre ligne est déjà sélectionnée par le module de commande, le procédé se poursuit préférentiellement par une troisième décision Q3 ou directement par une sixième étape 206 selon laquelle le module de commande sélectionne la nouvelle ligne et désélectionne chaque autre ligne qui n'est pas directement contiguë à ladite nouvelle ligne. Le procédé 200 peut ensuite soit continuer en rebouclant sur la troisième étape 203, soit se terminer.

Ainsi, dans le procédé 200 selon le premier aspect de l'invention, au plus deux lignes contiguës sont simultanément sélectionnées. En effet, lors de la deuxième décision Q2 :
- soit la nouvelle ligne est une deuxième ligne, c'est-à-dire qu'une et une seule autre ligne, la première ligne, est déjà sélectionnée par le module de commande. Dans ce cas, la deuxième ligne est sélectionnée et la première ligne reste sélectionnée si elle est contiguë avec la deuxième ligne, sinon elle est désélectionnée. On obtient donc soit une seule ligne sélectionnée, soit deux lignes contiguës sélectionnées.
- Soit la nouvelle ligne est une troisième ligne car des première et deuxième lignes contiguës sont déjà sélectionnées par le module de commande. Dans ce cas, la troisième ligne est sélectionnée et les première et deuxième lignes restent sélectionnées si elles sont contiguës avec la troisième ligne, sinon elles sont désélectionnées. On obtient donc soit une seule ligne sélectionnée, la troisième ligne, si aucune des première et deuxième lignes n'est contiguë avec elle, soit deux lignes contiguës sélectionnées : les première et troisième lignes ou les deuxième et troisième lignes.

Selon la deuxième alternative illustrée à la figure 2b, le procédé 200 peut comporter, si la réponse à la question de la deuxième décision Q2, « la ligne sélectionnée par l'opérateur est-elle une première ligne ? », est « non », c'est-à-dire si au moins une autre ligne est déjà sélectionnée par le module de commande, une troisième décision Q3 selon laquelle le module de commande répond à la question : « la ligne sélectionnée par l'opérateur est-elle une deuxième ligne ? ». Si oui, le procédé 200 se poursuit avec la sixième étape 206 précédemment décrite : le module de commande sélectionne la deuxième ligne et désélectionne la première ligne si elle n'est pas contiguë avec la deuxième ligne. Le procédé 200 peut ensuite soit continuer en rebouclant sur la troisième étape 203, soit se terminer.

Sinon, c'est-à-dire si la ligne sélectionnée par l'opérateur n'est pas une deuxième ligne donc est une troisième ligne, le procédé 200 se poursuit avec une septième étape selon laquelle la troisième ligne est sélectionnée et les première et deuxième lignes sont désélectionnées, même si l'une ou l'autre des première et deuxième lignes est contiguë de la troisième ligne.

Dans le cadre de la présente demande, une modification d'au moins une caractéristique d'aspect d'une ligne peut notamment être :
- un changement et notamment une augmentation d'épaisseur de ladite ligne, et/ou
- un changement de type de trait de ladite ligne, qui passe par exemple d'un trait continu à un trait pointillé ou d'un trait simple à un trait double, et/ou
- un changement de couleur de ladite ligne, et/ou
- un changement de dimensions de ladite ligne, par exemple de manière qu'elle dépasse de part et d'autre du cadran par rapport aux lignes non sélectionnées, et/ou
- un surlignage de ladite ligne, et/ou
- un ajout d'une marque à une extrémité de ladite ligne, par exemple entourer le chiffre à ladite extrémité, etc.

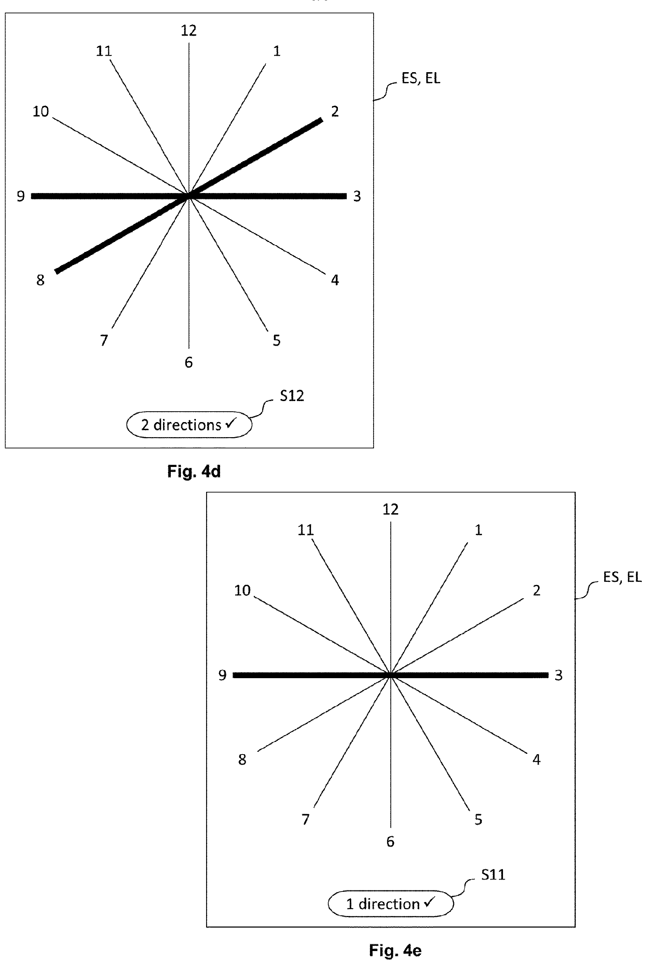

Les figures 4a, 4b, 4c, 4d et 4e montrent un exemple d'une succession de différents affichages réalisés par le module de commande.
- Sur la figure 4a, aucune ligne n'est sélectionnée et le premier symbole de validation S10 correspondant est affiché. Il s'agit par exemple de l'affichage de la deuxième étape 202.
- Puis, sur la figure 4b, l'opérateur sélectionne une première ligne : par exemple la première direction 1-7. Le module de commande valide la sélection de l'opérateur et modifie l'aspect de la première ligne. Le module de commande affiche préférentiellement le deuxième symbole de validation S11.
- Puis, sur la figure 4c, l'opérateur sélectionne une deuxième ligne : par exemple la deuxième direction 2-8 qui est contiguë à la première direction 1-7. Le module de commande valide la sélection de l'opérateur et modifie l'aspect de la deuxième ligne. Le module de commande affiche préférentiellement le troisième symbole de validation S12.
- Puis l'opérateur sélectionne une troisième ligne : par exemple la troisième direction 3-9 qui est contiguë à la deuxième direction 2-8. La figure 4d montre l'alternative conformément à la figure 2a selon laquelle le module de commande valide la sélection de l'opérateur, modifie l'aspect de la troisième ligne et désélectionne la première ligne, ici la première direction 1-7, qui n'est pas contiguë à la troisième ligne. Le module de commande affiche préférentiellement le troisième symbole de validation S12. La figure 4e montre l'alternative conformément à la figure 2b selon laquelle le module de commande valide la sélection de l'opérateur, modifie l'aspect de la troisième ligne et désélectionne les première et deuxième lignes, qu'elles soient contiguës à la troisième ligne ou non.

### Fin du procédé

Le procédé 200 selon le premier aspect de l'invention prend fin par une commande spécifique de l'opérateur, en interaction avec l'écran de sélection ES et le module de commande. La commande spécifique est par exemple :
- une commande tactile sur l'écran de sélection ES ;
- une commande via un périphérique connecté avec l'écran de sélection ES, comme une souris, un clavier, une manette directionnelle ;
- une commande vocale ;
- une commande gestuelle via une position ou un mouvement de la main.
Ces différents exemples de moyens d'interaction sont détaillés dans la suite.

Dans le mode préférentiel selon lequel le module de commande affiche le premier, deuxième ou troisième symbole de validation S10, S11, S12 en fonction du nombre de lignes sélectionnées, la commande spécifique est une action de sélection dudit premier, deuxième ou troisième symbole de validation S10, S11, S12 affiché.

Dans un autre mode selon lequel le module de commande affiche un même symbole de validation quel que soit le nombre de lignes sélectionnées, la commande tactile est une action de sélection de ce symbole de validation.

Lorsque le module de commande reçoit ladite commande spécifique, il met fin au test visuel du procédé 200 et définit le résultat du test visuel à partir de l'affichage en cours sur lequel zéro, une ou deux lignes sont sélectionnées. Lorsqu'au moins une ligne est sélectionnée, le module de commande calcule préférentiellement une valeur d'axe de cylindre compensateur - soit à partir de l'unique ligne sélectionnée, soit à partir de la bissectrice des deux lignes sélectionnées. Le module de commande peut ensuite afficher la valeur d'axe de cylindre compensateur calculée, sur l'écran de sélection ES uniquement ou à la fois sur l'écran de sélection ES et sur l'écran de lecture EL, et/ou la fournir en entrée d'un algorithme.

### Module de commande selon le deuxième aspect de l'invention

Le module de commande selon le deuxième aspect de l'invention comporte quant à lui des moyens pour afficher simultanément sur l'écran de sélection ES et sur l'écran de lecture EL la pluralité de lignes et préférentiellement l'un des premier, deuxième ou troisième symboles de validation S10, S11, S12.

Le module de commande selon le deuxième aspect de l'invention comporte également des moyens pour recevoir une sélection d'une ligne de la pluralité de lignes sur l'écran de sélection ES ainsi que des moyens pour recevoir une commande spécifique mettant fin au procédé 200 selon le premier aspect de l'invention.

Enfin, le support est préférentiellement une tête de réfracteur automatique et dans ce cas, le module de commande selon le deuxième aspect de l'invention comporte préférentiellement des moyens pour assurer la fonction de console de commande de la tête de réfracteur automatique, en particulier pour agencer un verre optique dans un emplacement de la tête de réfracteur automatique.

Par ailleurs, les moyens d'affichage sur l'écran de sélection ES et l'écran de lecture EL et de réception d'une sélection sur l'écran de sélection ES sont tels que :
- lorsque le module de commande reçoit une sélection d'une première ligne alors qu'aucune autre ligne n'est déjà sélectionnée, le module de commande sélectionne ladite première ligne ;
- lorsque le module de commande reçoit une sélection d'une nouvelle ligne alors qu'au moins une autre ligne est déjà sélectionnée, le module de commande sélectionne ladite nouvelle ligne, et le module de commande désélectionne chaque ligne qui n'est pas directement contiguë à ladite nouvelle ligne sélectionnée.

### Ecran de sélection et écran de lecture

L'opérateur interagit avec l'écran de sélection ES pour sélectionner une ligne ou réaliser la commande spécifique mettant fin au procédé 200 selon le premier aspect de l'invention. Le sujet examiné interagit quant à lui avec l'écran de lecture pour identifier si, à travers le support S, une ou plusieurs lignes affichées lui apparaissent plus nettes.

L'écran de sélection ES et l'écran de lecture EL peuvent être deux écrans distincts ou alternativement, un seul et même écran. L'affichage de la pluralité de lignes, avec le cas échéant la ou les lignes sélectionnées d'aspect modifié, est identique sur l'écran de sélection ES et sur l'écran de lecture EL de manière que l'opérateur et le sujet examiné accèdent à la même information. L'affichage du premier, deuxième ou troisième symbole de sélection S10, S11 ou S12, lorsqu'il a lieu, est réalisé au moins sur l'écran de sélection ES et préférentiellement à la fois sur l'écran de sélection ES et sur l'écran de lecture EL de manière que l'opérateur et le sujet examiné accèdent à la même information.

### Opérateur et sujet examiné

On définit l'opérateur comme étant celui qui interagit avec l'écran de sélection ES et le module de commande. L'opérateur est typiquement un praticien, tel qu'un ophtalmologue, un opticien ou même, dans le cadre de la présente invention, un assistant sans compétences particulières en physique de la réfraction et physiologie du système visuel. On définit le sujet examiné comme étant celui qui regarde l'écran de lecture à travers le support S. Le sujet examiné est typiquement un patient ou client du praticien.

Le sujet examiné peut lui-même être opérateur. Le sujet examiné peut être l'unique opérateur en l'absence de tout praticien. Alternativement, deux opérateurs distincts, par exemple le sujet examiné et un praticien, peuvent se relayer. Lorsque le sujet examiné est opérateur, soit l'écran de lecture forme un seul et même écran avec l'écran de sélection, soit l'écran de lecture est un deuxième écran de sélection.

### Système selon le troisième aspect de l'invention

Le système selon le troisième aspect de l'invention comporte le module de commande selon le deuxième aspect de l'invention ainsi que l'écran de sélection ES et l'écran de lecture EL.

L'écran de sélection ES peut être intégré avec le module de commande. C'est par exemple le cas d'un module de commande sous forme de tablette tactile ou d'ordinateur portable. Alternativement, l'écran de sélection ES peut être distinct du module de commande. C'est par exemple le cas d'un module de commande connecté avec un écran distant de type écran d'ordinateur, écran de télévision ou écran de projection.

L'écran de sélection ES et l'écran de lecture EL peuvent être un seul et même écran ou deux écrans distincts.

### Moyens d'interaction

Chaque étape de sélection d'une ligne et chaque commande spécifique pour mettre fin au procédé, le cas échéant en sélectionnant un symbole de validation, est réalisée par l'opérateur réalisant une action en interaction avec l'écran de sélection ES et le module de commande. Chaque action, de sélection d'une ligne ou de commande spécifique, peut être réalisée de différentes manières, dont plusieurs exemples non limitatifs sont listés ci-après.

L'action est préférentiellement réalisée au moyen d'un écran de sélection ES tactile : l'opérateur touche une zone de l'écran sur laquelle se trouve une ligne ou un symbole de validation afin de sélectionner ladite ligne ou ledit symbole de validation.

Alternativement, l'action peut être réalisée au moyen d'une interface qui est par exemple
- de type souris : l'opérateur positionne le curseur de la souris sur la zone de l'écran sur laquelle se trouve la ligne ou le symbole de validation et clique sur cette zone afin de sélectionner ladite ligne ou ledit symbole de validation ; ou
- de type clavier : chaque ligne est identifiée par une touche ou combinaison de touches qui lui est propre, par exemple la touche « 1 » pour la première direction 1-7, la touche « 2 » pour la deuxième direction 2-8, la touche « 3 » pour la troisième direction 3-9, etc. Une seule et même touche ou combinaison de touches, par exemple la touche « entrée », réalise avantageusement la commande spécifique. L'opérateur appuie sur une touche ou combinaison de touches afin de sélectionner la ligne correspondante ou de réaliser la commande spécifique ; ou
- de type manette directionnelle ou joystick : chaque ligne est identifiée par une direction qui lui est propre. La manette directionnelle comporte également un bouton associé à la commande spécifique. L'opérateur oriente la manette directionnelle dans une direction donnée afin de sélectionner la ligne correspondante, ou appuie sur le bouton afin de réaliser la commande spécifique.

Selon une autre alternative, l'action est réalisée au moyen d'une commande vocale, via un équipement de type microphone et un module de traitement du signal adapté dans le module de commande : chaque ligne est identifiée par un mot ou groupe de mots qui lui est propre, par exemple le mot « un » pour la première direction 1-7, le mot « deux » pour la deuxième direction 2-8, le mot « trois » pour la troisième direction 3-9, etc. Un seul et même mot ou groupe de mot, par exemple le mot « OK » ou « valider », réalise avantageusement la commande spécifique. L'opérateur prononce un mot ou groupe de mots afin de sélectionner la ligne correspondante ou de réaliser la commande spécifique.

Selon encore une autre alternative, la sélection est réalisée au moyen d'une commande gestuelle, via un équipement de type caméra et un module de traitement du signal adapté dans le module de commande. L'opérateur utilise un doigt, par exemple son index, comme un pointeur afin de désigner la zone de l'écran sur laquelle se trouve la ligne ou le symbole de validation qu'il souhaite sélectionner.

Les différentes actions de sélection du procédé 200 selon le premier aspect de l'invention peuvent être toutes réalisées d'une seule et même manière, par exemple au moyen d'un écran de sélection tactile. Alternativement, une combinaison de plusieurs techniques différentes peut être utilisée pour réaliser les différentes actions de sélection du procédé 200, par exemple les actions de sélection d'une ligne via un écran de sélection tactile ou via une commande gestuelle, et les actions de sélection d'un symbole de validation via une commande vocale, etc.

## Revendications

1. Procédé (200) de test visuel comportant une étape d'agencement d'un verre optique dans un emplacement d'un support et les étapes suivantes selon lesquelles un module de commande :
- affiche (202) sur un écran de sélection (ES) et sur un écran de lecture (EL) une pluralité de lignes, chaque ligne s'étendant dans une direction distincte, l'écran de sélection (ES) et l'écran de lecture (EL) étant deux écrans distincts ou alternativement un seul et même écran ;
- reçoit (203) une sélection d'une ligne sur l'écran de sélection (ES) ;
le procédé (200) étant tel que :
- lorsque (205) le module de commande reçoit une sélection d'une première ligne alors qu'aucune autre ligne n'est déjà sélectionnée, le module de commande sélectionne ladite première ligne en modifiant au moins une caractéristique d'aspect de ladite première ligne de manière qu'elle passe d'un aspect initial à un aspect modifié qui la différencie des lignes non sélectionnées ;
**caractérisé en ce que** :
- lorsque (206) le module de commande reçoit une sélection d'une nouvelle ligne alors qu'au moins une autre ligne est déjà sélectionnée, le module de commande sélectionne ladite nouvelle ligne en modifiant au moins une caractéristique d'aspect de ladite nouvelle ligne de manière qu'elle passe d'un aspect initial à un aspect modifié qui la différencie des lignes non sélectionnées, et le module de commande désélectionne en lui redonnant son aspect initial chaque ligne qui n'est pas directement contiguë à ladite nouvelle ligne sélectionnée.

2. Procédé (200) selon la revendication précédente **caractérisé en ce que** lorsque (204) le module de commande reçoit une sélection d'une ligne qui est déjà sélectionnée, le module de commande désélectionne ladite ligne en lui redonnant son aspect initial.

3. Procédé (200) selon l'une quelconque des revendications précédentes **caractérisé en ce que** lorsque (207), des première et deuxième lignes étant déjà sélectionnées, le module de commande reçoit une sélection d'une nouvelle ligne qui est une troisième ligne, le module de commande :
- sélectionne ladite troisième ligne en modifiant au moins une caractéristique d'aspect de ladite troisième ligne de manière qu'elle passe d'un aspect initial à un aspect modifié qui la différencie des lignes non sélectionnées, et
- désélectionne lesdites première et deuxième lignes en leur redonnant leur aspect initial.

4. Procédé (200) selon l'une quelconque des revendications précédentes **caractérisé en ce que** le module de commande affiche sur l'écran de sélection(ES) un symbole de validation en même temps que la pluralité de lignes.

5. Procédé (200) selon la revendication précédente **caractérisé en ce que** le symbole de validation est un premier symbole de validation tant qu'aucune ligne n'est sélectionnée, un deuxième symbole de validation distinct du premier symbole de validation lorsqu'une seule ligne est sélectionnée et un troisième symbole de validation distinct des premier et deuxième symboles de validation lorsque deux lignes sont sélectionnées.

6. Procédé (200) selon l'une quelconque des revendications précédentes **caractérisé en ce que** le module de commande calcule une valeur d'axe de cylindre compensateur à partir de la ou des lignes sélectionnée(s) et affiche cette valeur sur l'écran de sélection (ES) ou alternativement fournit cette valeur en entrée d'un algorithme.

7. Module de commande pour la mise en œuvre du procédé (200) de test visuel selon l'une quelconque des revendications précédentes, le module de commande ayant :
- des moyens pour afficher sur un écran de sélection (ES) et sur un écran de lecture (EL) une pluralité de lignes, chaque ligne s'étendant dans une direction distincte, l'écran de sélection (ES) et l'écran de lecture (EL) étant deux écrans distincts ou alternativement un seul et même écran ;
- des moyens pour recevoir une sélection d'une ligne de la pluralité de lignes sur l'écran de sélection (ES) ;
- des moyens pour recevoir une commande spécifique mettant fin au procédé (200) de test visuel ;
les moyens d'affichage sur l'écran de sélection (ES) et l'écran de lecture (EL) et de réception d'une sélection sur l'écran de sélection (ES) étant tels que :
- lorsque le module de commande reçoit une sélection d'une première ligne alors qu'aucune autre ligne n'est déjà sélectionnée, le module de commande sélectionne ladite première ligne en modifiant au moins une caractéristique d'aspect de ladite première ligne de manière qu'elle passe d'un aspect initial à un aspect modifié qui la différencie des lignes non sélectionnées ;
**caractérisé en ce que** :
- lorsque le module de commande reçoit une sélection d'une nouvelle ligne alors qu'au moins une autre ligne est déjà sélectionnée, le module de commande sélectionne ladite nouvelle ligne en modifiant au moins une caractéristique d'aspect de ladite nouvelle ligne de manière qu'elle passe d'un aspect initial à un aspect modifié qui la différencie des lignes non sélectionnées, et le module de commande désélectionne en lui redonnant son aspect initial chaque ligne qui n'est pas directement contiguë à ladite nouvelle ligne sélectionnée.

8. Système pour la mise en œuvre d'un procédé (200) de test visuel selon l'une quelconque des revendications 1 à 6, comportant le module de commande selon la revendication 7 ainsi que l'écran de sélection (ES) et l'écran de lecture (EL).

9. Système selon la revendication précédente **caractérisé en ce que** l'écran de sélection (ES) et l'écran de lecture (EL) sont deux écrans distincts.

10. Système selon la revendication 8 **caractérisé en ce que** l'écran de sélection (ES) et l'écran de lecture (EL) sont un seul et même écran.

## Patentansprüche

1. Visuelles Testverfahren (200), umfassend einen Anordnungsschritt eines optischen Glases an einer Stelle eines Trägers und die folgenden Schritte, gemäß denen ein Steuermodul:
- auf einem Auswahlbildschirm (ES) und auf einem Lesebildschirm (EL) eine Vielzahl von Zeilen, wobei sich jede Zeile anzeigt (202) in einer unterschiedlichen Richtung erstreckt, wobei der Auswahlbildschirm (ES) und der Lesebildschirm (EL) zwei unterschiedliche Bildschirme oder alternativ ein und derselbe Bildschirm sind,
- eine Auswahl einer Zeile auf dem Auswahlbildschirm (ES) empfängt (203);
wobei das Verfahren (200) derart ist, dass:
- wenn (205) das Steuermodul eine Auswahl aus einer ersten Zeile empfängt, während keinerlei andere Zeile bereits ausgewählt ist, das Steuermodul die genannte erste Zeile auswählt, indem es wenigstens ein Erscheinungsmerkmal der genannten ersten Zeile derart abändert, dass sie von einem ursprünglichen Erscheinungsbild zu einem abgeänderten Erscheinungsbild übergeht, das sie von den nicht ausgewählten Zeilen unterscheidet,
**dadurch gekennzeichnet, dass**:
- wenn (206) das Steuermodul eine Auswahl einer neuen Zeile empfängt, während wenigstens eine andere Zeile bereits ausgewählt ist, das Steuermodul die genannte neue Zeile auswählt, indem es wenigstens ein Erscheinungsbildmerkmal der genannten neuen Zeile derart abändert, dass sie von einem ursprünglichen Erscheinungsbild zu einem abgeänderten Erscheinungsbild übergeht, das sie von den nicht ausgewählten Zeilen unterscheidet, und das Steuermodul, indem es ihr wieder ihr ursprüngliches Erscheinungsbild zurückgibt, jede Zeile abwählt, die nicht mehr direkt an der genannten ausgewählten neuen Zeile anliegt.

2. Verfahren (200) gemäß dem voranstehenden Anspruch, **dadurch gekennzeichnet, dass**, wenn (204) das Steuermodul eine Auswahl einer Zeile empfängt, die bereits ausgewählt ist, das Steuermodul die genannte Zeile abwählt, indem sie ihr ihr ursprüngliches Erscheinungsbild zurückgibt.

3. Verfahren (200) gemäß irgendeinem der voranstehenden Ansprüche, **dadurch gekennzeichnet, dass**, wenn (207) die erste und die zweite Zeile bereits ausgewählt sind, das Steuermodul eine Auswahl einer neuen Zeile empfängt, die eine dritte Zeile ist, das Steuermodul:
- die genannte dritte Zeile auswählt, indem es wenigsten ein Erscheinungsbildmerkmal der genannten dritten Zeile derart abändert, dass sie von einem ursprünglichen Erscheinungsbild zu einem abgeänderten Erscheinungsbild übergeht, das sie von nicht ausgewählten Zeilen unterscheidet, und
- die genannte erste und die zweite Zeile abwählt, indem es ihnen ihr ursprüngliches Erscheinungsbild zurückgibt.

4. Verfahren (200) gemäß irgendeinem der voranstehenden Ansprüche, **dadurch gekennzeichnet, dass** das Steuermodul auf dem Auswahlbildschirm (ES) ein Bestätigungssymbol gleichzeitig mit der Vielzahl von Zeilen anzeigt.

5. Verfahren (200) gemäß dem voranstehenden Anspruch, **dadurch gekennzeichnet, dass** das Bestätigungssymbol ein erstes Bestätigungssymbol, solange keine Zeile ausgewählt ist, ein zweites, vom ersten Bestätigungssymbol unterschiedliches Bestätigungssymbol, wenn eine Zeile ausgewählt ist, und ein drittes, von dem ersten und dem zweiten Bestätigungssymbol unterschiedliches Bestätigungssymbol, ist, wenn zwei Zeilen ausgewählt sind.

6. Verfahren (200) gemäß irgendeinem der voranstehenden Ansprüche, **dadurch gekennzeichnet, dass** das Steuermodul einen Wert einer zylindrischen Ausgleichsachse ausgehend von der oder den ausgewählten Zeile(n) berechnet und diesen Wert auf dem Auswahlbildschirm (ES) anzeigt oder alternativ diesen Wert am Eingang eines Algorithmus liefert.

7. Steuermodul für die Umsetzung des visuellen Testverfahrens (200) gemäß irgendeinem der voranstehenden Ansprüche, wobei das Steuer-modul aufweist:
- Mittel zum Anzeigen einer Vielzahl von Zeilen auf einem Auswahlbildschirm (ES) und auf einem Lesebildschirm (EL), wobei sich jede Zeile in einer unterschiedlichen Richtung erstreckt, wobei der Auswahlbildschirm (ES) und der Lesebildschirm (EL) zwei unterschiedliche Bildschirme oder alternativ ein und derselbe Bildschirm sind;
- Mittel zum Empfangen einer Auswahl einer Zeile aus der Vielzahl von Zeilen auf dem Auswahlbildschirm (ES);
- Mittel zum Empfangen eines spezifischen Befehls, der das visuelle Testverfahren (200) beendet;
wobei die Mittel zum Anzeigen auf dem Auswahlbildschirm (ES) und dem Lesebildschirm (EL) und zum Empfangen einer Auswahl auf dem Auswahlbildschirm (ES) derart sind, dass:
- wenn das Steuermodul eine Auswahl einer ersten Zeile empfängt, dann keinerlei Zeile bereits ausgewählt ist, das Steuermodul die genannte erste Zeile auswählt, indem sie wenigstens ein Erscheinungsbildmerkmal genannten der ersten Zeile bereits derart abändert, dass sie von einem ursprünglichen Erscheinungsbild zu einem abgeänderten Erscheinungsbild übergeht, das sie von nicht ausgewählten Zeilen unterscheidet;
**dadurch gekennzeichnet, dass**:
- wenn das Steuermodul eine Auswahl einer neuen Zeile empfängt, während wenigstens eine andere Zeile ausgewählt ist, das Steuermodul die genannte neue Zeile auswählt, indem sie wenigstens ein Erscheinungsbildmerkmal der genannten neuen Zeile derart abändert, dass sie von einem ursprünglichen Erscheinungsbild zu einem abgeänderten Erscheinungsbild übergeht, das sie von nicht ausgewählten Zeilen unterscheidet, und das Steuermodul jede Zeile abwählt, die nicht direkt an der genannten neuen ausgewählten Zeile anliegt, indem es ihr ihr ursprüngliches Erscheinungsbild zurückgibt.

8. System für die Umsetzung eines visuellen Testverfahrens (200) gemäß irgendeinem der Ansprüche 1 bis 6, umfassend das Steuermodul gemäß Anspruch 7, sowie den Auswahlbildschirm (ES) und den Lesebildschirm (EL).

9. System gemäß dem voranstehenden Anspruch, **dadurch gekennzeichnet, dass** der Auswahlbildschirm (ES) und der Lesebildschirm (EL) zwei unterschiedliche Bildschirme sind.

10. System gemäß Anspruch 8, **dadurch gekennzeichnet, dass** der Auswahlbildschirm (ES) und der Lesebildschirm (EL) ein und derselbe Bildschirm sind.

## Claims

1. Method (200) for eyesight testing comprising a step of arranging an optical glass in a location of a support and the following steps according to which a control module:
- displays (202) on a selection screen (ES) and on a reading screen (EL) a plurality of lines, each line extending in a distinct direction, the selection screen (ES) and the reading screen (EL) being two distinct screens or alternatively a single and same screen;
- receives (203) a selection of a line on the selection screen (ES);
the method (200) being such that:
- when (205) the control module receives a selection of a first line while no other line has been selected yet, the control module selects said first line by modifying at least one appearance-related feature of the first line so that the first line changes from an initial appearance to a modified appearance that differentiates it from the lines that are not selected;
**characterised in that**:
- when (206) the control module receives a selection of a new line while at least one other line has already been selected, the control module selects said new line by modifying at least one appearance-related feature of the new line so that the new line changes from an initial appearance to a modified appearance that differentiates it from the lines that are not selected, and the control module deselects each line that is not directly adjacent to said new selected line by giving each of said non-adjacent lines its initial appearance.

2. Method (200) according to the preceding claim **characterised in that** when (204) the control module receives a selection of a line which is already selected, the control module deselects said line by giving it its initial appearance.

3. Method (200) according to any preceding claim **characterised in that** when (207), with first and second lines being already selected, the control module receives a selection of a new line which is a third line, the control module:
- selects said third line by modifying at least one appearance-related feature of said third line so that the third line changes from an initial appearance to a modified appearance that differentiates it from the lines that are not selected, and
- deselects said first and second lines by giving them their aspect initial.

4. Method (200) according to any preceding claim **characterised in that** the control module displays on the selection screen (ES) a validation symbol at the same time as the plurality of lines.

5. Method (200) according to the preceding claim **characterised in that** the validation symbol is a first validation symbol as long as no line has been selected, a second validation symbol distinct from the first validation symbol when a single line is selected and a third validation symbol distinct from the first and second validation symbols when two lines are selected.

6. Method (200) according to any preceding claim **characterised in that** the control module calculates a compensating cylinder axis value from the selected line or lines and displays this value on the selection screen (ES) or alternatively provides this value as input of an algorithm.

7. Control module for the implementation of the eyesight testing method (200) according to any preceding claim, the control module having:
- means for displaying on a selection screen (ES) and on a reading screen (EL) a plurality of lines, each line extending in a distinct direction, the selection screen (ES) and the reading screen (EL) being two distinct screens or alternatively a single and same screen;
- means for receiving a selection of a line of the plurality of lines on the selection screen (ES);
- means for receiving a specific command putting an end to the eyesight testing method (200);
the means for displaying on the selection screen (ES) and the reading screen (EL) and for receiving a selection on the selection screen (ES) being such that:
- when the control module receives a selection of a first line while no other line has been selected yet, the control module selects said first line by modifying at least one appearance-related feature of the first line so that the first line changes from an initial appearance to a modified appearance that differentiates it from the lines that are not selected;
**characterised in that**:
- when the control module receives a selection of a new line while at least one other line has already been selected, the control module selects said new line by modifying at least one appearance-related feature of the new line so that the new line changes from an initial appearance to a modified appearance that differentiates it from the lines that are not selected, and the control module deselects each line that is not directly adjacent to said new selected line by giving each of said non-adjacent lines its initial appearance.

8. System for the implementation of an eyesight testing method (200) according to any of claims 1 to 6, comprising the control module according to claim 7 as well as the selection screen (ES) and the reading screen (EL).

9. System according to the preceding claim **characterised in that** the selection screen (ES) and the reading screen (EL) are two distinct screens.

10. System according to claim 8 **characterised in that** the selection screen (ES) and the reading screen (EL) are a single and same screen.
